(19) Europäisches Patentamt / European Patent Office / Office européen des brevets

(11) **EP 2 303 770 B1**

(12) **EUROPEAN PATENT SPECIFICATION**

(45) Date of publication and mention
of the grant of the patent:
**09.07.2014 Bulletin 2014/28**

(21) Application number: **09774224.1**

(22) Date of filing: **29.06.2009**

(51) Int Cl.:
*B82B 3/00* (2006.01)          *D06M 16/00* (2006.01)
*D06M 10/00* (2006.01)      *D06M 23/08* (2006.01)
*A61L 2/238* (2006.01)        *C08J 7/06* (2006.01)
*B01D 67/00* (2006.01)        *B01D 69/14* (2006.01)
*A01N 59/16* (2006.01)        *B22F 1/00* (2006.01)
*B22F 9/02* (2006.01)          *D06M 10/06* (2006.01)
*D06M 11/83* (2006.01)

(86) International application number:
**PCT/US2009/049026**

(87) International publication number:
**WO 2010/002773 (07.01.2010 Gazette 2010/01)**

(54) **METHOD FOR IN SITU FORMATION OF METAL NANOCLUSTERS WITHIN A POROUS SUBSTRATE**

VERFAHREN ZUR IN-SITU-BILDUNG VON METALLNANOCLUSTERN IN EINEM PORÖSEN SUBSTRAT

PROCÉDÉ POUR LA FORMATION IN SITU DE NANOAGRÉGATS À L'INTÉRIEUR D'UN SUBSTRAT POREUX

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **30.06.2008 US 77080**

(43) Date of publication of application:
**06.04.2011 Bulletin 2011/14**

(73) Proprietor: **3M Innovative Properties Company Saint Paul, MN 55133-3427 (US)**

(72) Inventor: **WEISS, Douglas, E.
Saint Paul, MN 55133-3427 (US)**

(74) Representative: **Vossius & Partner
Siebertstrasse 4
81675 München (DE)**

(56) References cited:
**US-A1- 2007 044 590       US-A1- 2007 134 902**

• **DATABASE WPI Week 200579 Thomson Scientific, London, GB; AN 2005-773599 XP002674932, & JP 2005 298993 A (HOWA KK) 27 October 2005 (2005-10-27)**
• **HE J. ET AL: 'Facile In Situ Synthesis of Noble Metal Nanoparticles in Porous Cellulose Fibers' CHEMISTRY OF MATERIALS vol. 15, 2003, pages 4401 - 4406, XP002981439**
• **HORNEBECQ V. ET AL: 'Stable Silver Nanoparticles Immobilized in Mesoporous Silica' CHEMISTRY OF MATERIALS vol. 15, 2003, pages 1993 - 1999, XP008141424**
• **RAMNANI S.P. ET AL: 'Synthesis of silver nanoparticles supported on silica aerogel using gamma radiolysis' RADIATION PHYSICS AND CHEMISTRY vol. 76, 2007, pages 1290 - 1294, XP022094637**
• **CHEN C. ET AL: 'In-Situ Formation of Silver Nanoparticles on Poly(N-isopropylacrylamide)-Coated Polystyrene Microspheres' ADVANCED MATERIALS vol. 10, no. 14, 1998, pages 1122 - 1126, XP000781878**

**Description**

**FIELD**

This invention relates to a continuous method for *in situ* formation of metal nanoclusters within a porous substrate. The articles comprising porous substrates containing metal nanoclusters are useful, for example, in antimicrobial application.

**BACKGROUND**

Some metals such as silver, gold, platinum, and palladium exhibit antimicrobial activity. Silver, for example, has been well known for its antimicrobial effects for centuries. In recent years, various products containing silver have been introduced into the marketplace for antimicrobial uses, for example, in wound care, respirators, textiles, membranes, filter media, powder coatings, and moldable plastics. Various methods have been employed to provide silver to the articles. For example, in many cases, silver nanoparticles are first made or purchased and then deposited (for example, by sputtering or ion deposition) onto the article. In other cases, for example, silver is formed on the article by precipitating a silver salt or by complexing silver with an amine. In still other cases, for example, textile fibers are coated with silver and then used in knits, wovens, and non-wovens.

US 2007/044590 discloses a method for producing metal nanoparticles, comprising steps of (a) dissolving a metallic compound in a solvent to obtain a metal ionic solution; (b) uniformly distributing said metal ionic solution on a carrier; (c) shooting electrons from an electron source at said carrier for reducing said metal ions to metal nanoparticles.

JP 2005-298993 discloses a specific fiber structure which contains inorganic microparticles injected and dispersed in a fiber structure base material consisting of an organic macromolecular material.

Chem. Mater. 2003, 15, 4401 - 4406 relates to an *in situ* synthesis of noble metal nanoparticles in porous cellulose fibers.

Radiation Physics and Chemistry 2007, 76, 1290 - 1294 relates to a synthesis of silver nanoparticles supported on silica aerogel using gamma radiolysis.

Chem. Mater. 2003, 15, 1993 - 1999 relates to stable silver nanoparticles immobilized in mesoporous silica. >

**SUMMARY**

In view of the foregoing, we recognize that there is a need in the art for a method of providing nanosized metals in a porous substrate. Furthermore, we realize that it would be advantageous for such a method to be a simple method that can be carried out in a continuous manner without requiring vacuum or colloidal chemistry. Briefly, in one aspect, the present invention provides a continuous method for *in situ* forming metal nanoclusters within a porous substrate. The method comprises (a) imbibing a porous substrate with an aqueous heavy metal salt solution, and (b) exposing the imbibed porous substrate to ionizing radiation to form heavy metal nanoclusters within the porous substrate, wherein the method is carried out without any vacuum processing and the porous substrate is a fabric or a microporous substrate comprising one or more polymeric materials.

Surprisingly, the method of the invention provides for *in situ* formation of heavy metal nanoclusters within the porous substrate. There is no separate step required to first form the heavy metal nanoclusters before depositing them. Furthermore, it has been discovered that inducing heavy metal nanoclusters to nucleate in a porous substrate prevents the coalescence and growth of such nanoclusters into larger clusters. Thus, the size of resulting heavy metal nanoclusters is self-limited by the size of the interstices or pores of the substrate. Advantageously, the method of the invention is carried out in a continuous manner. In addition, it does not use any vacuum processing and does not require use of colloidal chemistry. The method of the invention therefore meets the need in the art for a simple method of providing nanosized metals in a porous substrate. In another aspect, articles comprising heavy metal nanoclusters imbedded within a porous substrate are disclosed.

As used herein, the term "heavy metal" refers to any element with an atomic number greater than 21 (scandium); and the term "nanocluster" refers to an individual particle or a cluster of agglomerated particles having dimensions of less than about 100 nm.

**DESCRIPTION OF THE DRAWINGS**

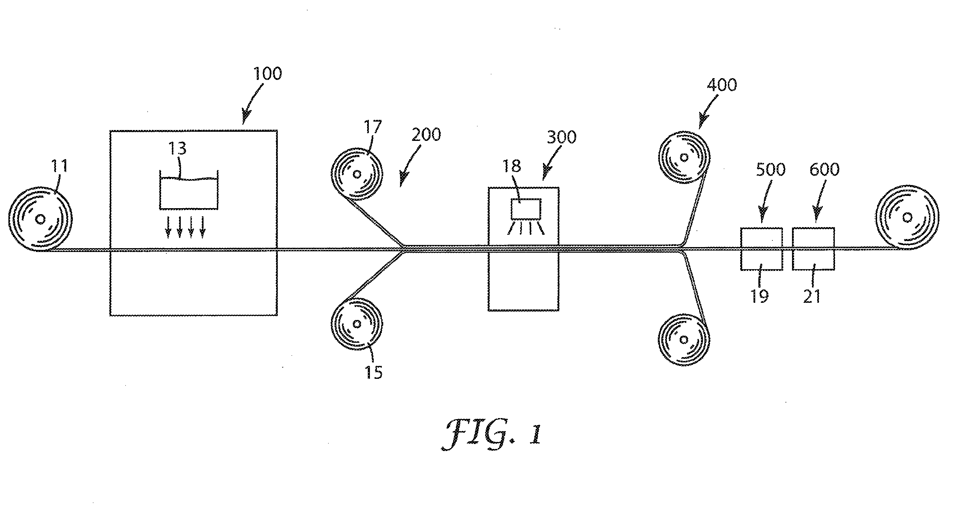

**Fig. 1** is a schematic representation of a method for forming metal nanoclusters within a porous substrate according to the present invention.

## DETAILED DESCRIPTION

In the method of the invention, the porous substrate serves as an isolation matrix during formation of the heavy metal nanoclusters. Any porous substrate having interstices or pores of desired size for the resulting heavy metal nanoclusters can be utilized as long as the substrate can withstand a mild dose of ionizing radiation. Typically, the porous substrate will have an effective pore size ranging from about 30 nm up to about 100 micrometers. The porous substrate is a fabric or a microporous substrate comprising one or more polymeric materials. The porous substrate can be hydrophilic or hydrophobic. The porous substrate can be charged as an electret (i.e. a dielectric substrate that exhibits a quasi-permanent electric charge).

In some embodiments, the porous substrate is a fabric. The fabric can be formed by conventional fabric forming processes such as weaving, knitting, braiding, and the like. Preferably, the fabric is a non-woven fabric. Suitable non-woven fabrics can be made by processes such as (a) melt blowing, (b) spunlaid, (c) spinning and (d) wet or dry laying a plexifilament formation or solution or melt nanofiber spinning. The formation of fine fiber fabrics can involve techniques such as fiber splitting (for example, by mechanical combing or other processing, or by water entanglement), and spun lace processing (for example, by water entanglement). It can be preferred for the fabric to have been formed by melt blowing.

In non-wovens, the interstices or pore size is typically at most 3 times the average fiber size and can typically be as small as 1.5 times the average fiber size if densified or coated.

In one embodiment, the porous substrate is a microporous substrate. Microporous substrates typically have an average pore size less than about 100 $\mu$m (preferably, less than about 10 $\mu$m; more preferably, less than about 1 $\mu$m). Suitable microporous substrates include, but are not limited to, microporous membranes, and microporous fibers.

Microporous substrates comprise one or more polymeric materials resulting in a hydrophobic microporous substrate. Suitable polymeric materials include, but are not limited to, polyolefins, poly(isoprenes), poly(butadienes), fluorinated polymers, chlorinated polymers, polyesters, polyamides, polyimides, polyethers, poly(ether sulfones), poly(sulfones), polyphenylene oxides, poly(vinyl acetate), copolymers of vinyl acetate, poly(phosphazenes), poly(vinyl esters), poly(vinyl ethers), poly(vinyl alcohols), and poly(carbonates). Suitable polyolefms include, but are not limited to, poly(ethylene), poly(propylene), poly(1-butene), copolymers of ethylene and propylene, alpha olefin copolymers (such as copolymers of 1-butene, 1-hexene, 1-octene, and 1-decene), poly(ethylene-co-1-butene) and poly(ethylene-co-1-butene-co-1-hexene). Suitable fluorinated polymers include, but are not limited to, poly(vinyl fluoride), poly(vinylidene fluoride), copolymers of vinylidene fluoride (such as poly(vinylidene fluoride-co hexafluoropropylene), and copolymers of chlorotrifluoroethylene (such as poly(ethylene-co-chlorotrifluoroethylene). Suitable polyamides include, but are not limited to, poly(imino(1-oxohexamethylene)), poly(iminoadipoyliminohexamethylene), poly(iminoadipoyliminodecamethylene), and polycaprolactam. Suitable polyimides include, but are not limited to, poly(pyromellitimide). Suitable poly(ether sulfones) include, but are not limited to, poly(diphenylether sulfone) and poly(diphenylsulfone-co-diphenylene oxide sulfone). Suitable copolymers of vinyl acetate include, but are not limited to, poly(ethylene-co-vinyl acetate) and such copolymers in which at least some of the acetate groups have been hydrolyzed to afford various poly(vinyl alcohols).

Suitable commercially available hydrophilic and hydrophobic microporous membranes are available under the trade designations DURAPORE(™) and MILLIPORE(™) EXPRESS MEMBRANE, available from Millipore Corporation of Billerica, Massachusetts. Other suitable commercial microporous membranes known under the trade designations NYLAFLO and SUPOR (™) are available from Pall Corporation of East Hills, New York.

The microporous substrate may contain inorganic fillers such as silica or titania.

In one desired embodiment, the microporous substrate is a hydrophobic microporous membrane comprising one or more of the above-mentioned polymeric materials. Preferably, the hydrophobic microporous membrane is in the form of a thermally-induced phase separation (TIPS) membrane. In other desired embodiments, the porous substrate is a hydrophilic microporous membrane such as a hydrophilic TIPS membrane.

TIPS membranes are often prepared by forming a solution of a thermoplastic material and a second material above the melting point of the thermoplastic material. Upon cooling, the thermoplastic material crystallizes and phase separates from the second material. The crystallized material is often stretched. The second material is optionally removed either before or after stretching. TIPS membranes and methods of making the same are disclosed in U.S. Patent Nos. 1,529,256; 4,539,256; 4,726,989; 4,867,881; 5,120,594; 5,260,360; and 5,962,544.

In some embodiments, TIPS membranes comprise polymeric materials such as poly(vinylidene fluoride) (PVDF), polyolefins such as poly(ethylene) or poly(propylene), vinyl-containing polymers or copolymers such as ethylene-vinyl alcohol copolymers and butadiene-containing polymers or copolymers, and acrylate-containing polymers or copolymers. TIPS membranes comprising PVDF are further described in U.S. Patent Application Publication No. 2005/0058821 (Smith et al.).

In another embodiment, the porous substrate is a non-woven web. As used herein, the term "non-woven web" refers to a fabric that has a structure of individual fibers or 5 filaments which are randomly and/or unidirectionally interlaid in a mat-like fashion.

Useful non-woven webs can be made by methods commonly known in the art. For example, a fibrous non-woven web can be made by carded, air-laid, spunlaced, spunbonding, or melt-blowing techniques or combinations thereof. Spunbonded fibers are typically small diameter fibers that are formed by extruding molten thermoplastic polymer as filaments from a plurality of fine, usually circular capillaries of a spinneret with the diameter of the extruded fibers being rapidly reduced. Meltblown fibers are typically formed by extruding the molten thermoplastic material through a plurality of fine, usually circular, die capillaries as molten threads or filaments into a high velocity, usually heated gas (for example, air) stream which attenuates the filaments of molten thermoplastic material to reduce their diameter. Thereafter, the meltblown fibers are carried by the high velocity gas stream and are deposited on a collecting surface to form a web of randomly disbursed meltblown fibers. Any of the non-woven webs may be made from a single type of fiber or two or more fibers that differ in the type of thermoplastic polymer and/or thickness.

Suitable non-woven webs include, for example, the melt-blown microfiber non-woven webs described in Wente, V.A., "Superfine Thermoplastic Fibers"; Industrial Engineering Chemistry, 48, 1342-1346 (1956), and Wente, V.A., "Manufacture of Super Fine Organic Fibers"; Naval Research Laboratories (Report No. 4364). May 25, 1954. For example, the non-woven web can be prepared from ethylene-vinyl alcohol copolymers as described in U.S. Patent No. 5,962,544. In some embodiments, suitable non-woven webs can be prepared from nylon.

The porous substrate is imbibed with an aqueous heavy metal salt solution. Preferably, the heavy metal salt solution comprises a gold salt, a silver salt, a palladium salt, a platinum salt, a copper salt, or a combination thereof (more preferably, a gold salt, a silver salt, a copper salt, or a combination thereof; most preferably, a gold salt, a silver salt, or a combination thereof).

Common heavy metal salts include, for example, heavy metal sulfates, phosphates, lactates, chlorides, bromides, carbonates, and nitrates. Preferably, the heavy metal salt is a sulfate.

Typically, the aqueous heavy metal salt solution comprises less than about 5 % by weight heavy metal salt. Preferably, the aqueous heavy metal salt solution comprises from about 0.01 % to about 1 % by weight heavy metal salt (more preferably, from about 0.2 % to about 1 % by weight heavy metal salt). The concentration of the aqueous heavy metal salt solution can influence, to some degree, the size of the resulting heavy metal nanoparticles, the number of heavy metal nanoparticles formed, and the dose of ionizing radiation needed to form the heavy metal nanoparticles.

Optionally, the aqueous heavy metal salt solution can further comprise a non-polar solvent such as, for example, alcohols, ketones, esters, or other organic solvents that are at least partially miscible with water. Non-polar solvent may be added for one or more reasons. For example, non-polar solvent may be added to improve wetting, to reduce solubility (that is, to promote precipitation), or to drive equilibrium toward formation of metals (that is, to promote metal particle formation). Typically, the non-polar solvent is added in an amount up to about 25 %.

Preferably, the non-polar solvent is an alcohol (more preferably, the non-polar solvent is isopropanol). Addition of a non-polar solvent is particularly useful in overcoming the surface energy of hydrophobic porous substrates.

The aqueous heavy metal salt solution can be imbibed into the porous substrate using coating methods known in the art such as, for example, dip coating, blade coating, roll coating, slot coating, gravure coating, slide coating, curtain coating, notch coating, spin coating, and the like. Preferably, the porous substrate is saturated with the aqueous heavy metal salt solution.

After the porous substrate is imbibed with the aqueous heavy metal salt solution, it is exposed to ionizing radiation to form heavy metal nanoclusters in the porous substrate. As used herein, "ionizing radiation" means radiation of a sufficient dosage and energy to reduce metal ions to metal clusters. The ionizing radiation can be, for example, electron-beam (e-beam), gamma radiation, or x-ray.

E-beam and gamma radiation are preferred for the method of the invention due, in part, to the availability of commercial sources. E-beam generators, for example, are commercially available from a variety of sources, including the ESI "ELECTROCURE" EB SYSTEM from Energy Sciences, Inc. (Wilmington, MA), and the BROADBEAM(™) EB PROCESSOR from PCT Engineered Systems, LLC (Davenport, IA). Sources of gamma irradiation are commercially available from MDS Nordion using a cobalt-60 high-energy source. For any given piece of equipment and irradiation sample location, the dosage delivered can be measured in accordance with ASTM E-1275 entitled "Practice for Use of a Radiochromic Film Dosimetry System." By altering extractor grid voltage, beam diameter and/or distance to the source, various dose rates can be obtained.

Radiation doses can be administered in a single dose of the desired level or in multiple doses which accumulate to the desired level. Dosages typically range cumulatively from about 10 kGy to about 40 kGy. For radiation sensitive substrates such as, for example, polypropylene, low doses of ionizing radiation can be used to avoid polymer degradation.

Heavy metal nanoclusters are directly formed and uniformly dispersed within the porous substrate. The porous substrate acts as an isolation matrix and self-limits the nanocluster size. The size of the heavy metal nanoclusters will therefore depend upon the size of the interstices or pores in the substrate and any post-irradiation techniques. Typically, the heavy metal nanoclusters will be less than 100 nm. In some embodiments the average size of the heavy metal nanoclusters is less than about 40 nm. In some preferred embodiments, the average size of the heavy metal nanoclusters is between about 20 nm and about 40 nm.

In order to tightly bind or fix the heavy metal nanoparticles in the porous substrate, the imbibed porous substrate can be dried after formation of the nanoparticles by exposure to ionizing radiation. Drying can be accomplished using methods known in the art including, for example, heating (for example, in an oven or using a heat gun), gap drying, gas (nitrogen) impingement, forced air, and the like. Preferably, the imbibed porous substrate is dried in a heated oven. The imbibed porous substrate can be heated up to any temperature that does not damage the substrate. Any conventional oven can be used. Suitable ovens include, but are not limited to, convection ovens.

After drying, the porous substrate with heavy metal nanoclusters imbedded within can optionally be washed (for example, to remove residual heavy metal salt) using methods known in the art. In some cases, water can be used to extract water soluble residual salts. If the dried porous substrate is hydrophobic, however, an alcohol/water solution will be needed to wet the substrate so that the residual salts can be removed. Depending upon the desired end use of the heavy metal nanoclusters, it may be desirable that the nanoclusters be leachable from the porous substrate. When leachable heavy metal nanoclusters are desired, the washing step can be carried out before optionally drying. A dispersion of heavy metal nanoclusters can then be obtained.

Alternatively, washing the imbibed porous substrate before drying can be utilized to redistribute the heavy metal nanoclusters throughout the substrate and thus improve the nanocluster distribution throughout the substrate. When the washing step is done immediately after irradiation, continued nanocluster growth is limited by dilution of the imbibing solution. The nanoclusters can be isolated in solution for some other application or, with time, are attracted back to the substrate and captured by it through columbic attraction. This can lead to a more uniform distribution of the originally formed clusters.

The method of the invention is carried out in a continuous manner. An exemplary continuous method for forming metal nanoclusters within a porous substrate according to the present invention is depicted in **Fig**. **1.** The exemplary method includes an imbibing step **100**, a lining step **200,** an irradiation step **300,** a liner removal step **400,** a drying step **500,** and a wash step **600.**

As shown in **Fig. 1,** a roll of porous substrate **11** can be unwound so that porous substrate **11** enters into the imbibing step **100.** The porous substrate **11** is exposed to aqueous heavy metal salt solution **13** for a desired amount of time to allow absorption.

Once the porous substrate **11** has been imbibed in the heavy metal salt solution **13,** one or more liners can be added to the imbibed porous substrate **11** in the lining step **200.** In **Fig. 1,** addition of an optional removable carrier liner **15** and an optional removable cover liner **17** is shown. Typical liner sheet materials include, but are not limited to, polyethylene terephthalate film materials, and other polymer film materials.

The imbibed porous substrate **11** then proceeds to the irradiation step **300** where it is exposed to a sufficient quantity of ionizing radiation using at least one device capable of providing a sufficient dose of radiation (preferably, e-beam or gamma radiation; more preferably, e-beam) **18** to reduce the heavy metal ion to heavy metal nanoclusters.

After the irradiation step **300,** the optional removable carrier liner **15** and optional removable cover liner **17** can be removed in the liner removal step **400.** The porous substrate **11,** which now contains heavy metal nanoclusters can then move on to the drying step **500.**

As depicted in **Fig. 1,** the drying step **500** can be carried out using an oven **19.** The drying step **500** fixes the heavy metal nanoclusters in the porous substrate **11.** The drying step **500** can be omitted when leachable heavy metal nanoclusters are desired.

Next, the porous substrate **11** containing heavy metal nanoclusters can be washed in the wash step **600** in a rinse chamber or with a rinse spray **21.** If the drying step **500** was omitted, the heavy metal nanoclusters can be leached out of the porous substrate **11** (or redistributed throughout porous substrate **11**) during the wash step **600.**

Following the wash step **600,** the porous substrate can be taken up in roll form. The porous substrate containing heavy metal nanoclusters can be stored for future use in roll form or used immediately.

Porous substrates comprising heavy metal nanoclusters imbedded in the porous substrate are useful in many applications such as, for example, antimicrobial applications, filtering applications (for example, water filtration, catalytic filters, cabin air filters, or respirators), electromagnetic field (EMF) shielding applications, and more.

For antimicrobial applications, the porous substrate comprises heavy metal nanoclusters of a heavy metal that exhibits antimicrobial activity such as, for example, silver, gold, copper, platinum, or palladium. Such articles are especially useful in applications such as water filtration, air filtration, wound care, and food packaging. Examples of antimicrobial articles that can be made with the articles of the invention include antimicrobial sponges, wipes, pads, filters, clothing, shoe liners, food packaging, bedding, and the like. Articles such as, for example clothing for military applications, can also provide resistance to bioagents.

The electret charge can be imparted to the (e.g. nonwoven) web articles using various known techniques such as hydrocharging, corona charging, and combinations thereof. Unlike an electrostatic charge that dissipates shortly thereafter (such as can be created as a result of friction), the electret charge of the (e.g. nonwoven) web articles is substantially maintained for the intended product life of the article. Hence, sufficient charge is evident at the time of use as well as at least 6 months or 12 months after manufacturing.

Surprisingly, it has been discovered that the method of the invention for *in situ* formation of metal nanoclusters within a porous substrate does not discharge the electret charge imparted on the substrate (that is, a charged substrate remains charged even after exposing the substrate to ionizing radiation).

**EXAMPLES**

Objects and advantages of this invention are further illustrated by the following examples, but the particular materials and amounts thereof recited in these examples, as well as other conditions and details, should not be construed to unduly limit this invention.

**Test Methods**

The following test methods were used to characterize the TIPS membranes and nonwovens used in the examples:

**Water Flux Measurement.** Water flux was determined by placing a disk of the test film or nonwoven having a diameter of approximately 47 millimeters (mm) (1.85 inches) in a Model 4238 Pall Gelman magnetic filter holder (available from Pall Corp., East Hills, NY). The filter holder was then placed on a filter flask that was attached to a vacuum pump. A vacuum gauge was used to monitor the vacuum. Approximately 150 milliliters (ml) of water was placed in the filter holder and then vacuum was applied. After approximately 50 ml of water had passed through the film (the vacuum gauge at this time indicated approximately 0.83 mm of mercury (approximately 21 inches of mercury), timing was commenced using a stopwatch. When all of the remaining water had passed through the film, timing was stopped. The water flux was the time, measured in seconds that elapsed for 100 ml of water to pass through the membrane under a vacuum of 0.83 mm of mercury. If 400 ml of water passed through the substrate before reaching the desired pressure, the flux was deemed too fast to measure at these conditions

**Gurley Porosity.** Gurley is a measure of the resistance to flow of a gas through a membrane, expressed as the time necessary for a given volume of gas to pass through a standard area of the membrane under standard conditions, as specified in ASTM D726-58, Method A. Gurley is the time in seconds for 50 cubic centimeters (cc) of air, or another specified volume, to pass through 6.35 $cm^2$ (one square inch) of the membrane at a pressure of 124 mm of water. The film sample was clamped between cylindrical rings, the uppermost of which contained a piston and the specified volume of air. When released, the piston applied pressure, under its own weight, to the air in the upper cylinder and the time for the specified volume of air to pass through the membrane was measured.

**Bubble Point.** The Bubble Point pore size is the bubble point value representing the largest effective pore size measured in microns according to ASTM-F-316-80.

The following test methods were used to characterize antimicrobial properties of the materials produced in the examples.

**Zone of Inhibition Test Method.** Suspensions were made in a test tube at an appropriate turbidity of the bacterial culture to be tested. A sterile cotton swab was placed in the bacterial suspension and excess fluid was removed by pressing and rotating the cotton swab against the inside of the tube above the fluid level. The swab was sweeped in at least three directions over the surface of Mueller-Hinton II agar to obtain uniform growth. A final sweep was made around the rim of the agar. The plates were dried for five minutes. A sample disk was placed on the plate using sterile forceps. The plates were incubated soon after placing the sample disks for the appropriate incubation time. After incubation, the diameter of the zone of growth inhibition around each disk was measured to the nearest whole mm. Plates were examined carefully for well-developed colonies within the zone of inhibition. The results listed include the size of the 7 mm disk and zone of inhibition.

**Assessment of Antibacterial Finishes on Textile Materials (AATCC Method 100).** Samples were tested in accordance with AATCC Method 100 using D/E Neutralizing broth and Petrifilm™ Aerobic Count Plates for enumeration. Samples were not sterilized prior to testing. Each sample was inoculated with 0.5 ml of a suspension containing approximately $1\text{-}2 \times 10^5$ colony forming units (cfu)/ml of an appropriate test organism. Separate sample swatches were inoculated for each time point desired (0 hour and 24 hours). The set of swatches for 24 hours were incubated at 28°C. Immediately after inoculation (0 hour), one swatch of sample was placed in a sterile stomacher bag and 100 ml of D/E Neutralizing Broth was added. The sample was processed for two minutes in a Seward Model 400 Stomacher. Serial dilutions of $10^0$, $10^1$ and $10^2$ and aerobic plate count using 3M Petrifilm™ Aerobic Count (AC) were performed. At 24 hour incubation time, samples were processed as above. Total colony forming units per sample were recorded after 48 hours of incubation of the Petrifilm™ at 35°C $\pm$ 1°C. The percent reduction in microbial numbers was calculated. Samples were tested against *Staphylococcus aureus* (ATCC 6538) and *Pseudomonas aeruginosa* (ATCC 9027). Two duplicates of each sample and at each time point were tested. The reported results below are the average of the two samples.

Pressure drop and Quality Factor measurements of electret charged non-woven webs:

The filtration performance of the nonwoven blown microfiber (BMF) webs were evaluated using an Automated Filter Tester AFT Model 8127 (available from TSI, Inc., St. Paul, MN) using dioctylphthalate (DOP) as the challenge aerosol and a MKS pressure transducer that measured pressure drop ($\Delta P$ (mm of $H_2O$)) across the filter. The DOP aerosol is nominally a monodisperse 0.3 micrometer mass median diameter having an upstream concentration of 70 - 120 mg/m$^3$. The aerosol was forced through a sample of filter media at a face velocity of 6.9 cm/s with the aerosol ionizer turned off. The total testing time was 23 seconds (rise time of 15 seconds, sample time of 4 seconds, and purge time of 4 seconds). The concentration of DOP aerosol was measured by light scattering both upstream and downstream of the filter media using calibrated photometers. The DOP % Penetration (% Pen) is defined as: % Pen = 100x(DOP concentration downstream/DOP concentration upstream). For each material, 6 separate measurements were made at different locations on the BMF web and the results were averaged. The % Pen and $\Delta P$ were used to calculate a Quality Factor (QF) by the following formula:

$$QF = - \ln(\% \text{ Pen}/100)/ \Delta P,$$

where In stands for the natural logarithm. A higher QF value indicates better filtration performance and decreased QF values effectively correlate with decreased filtration performance

Initial Filtration Performance:

Each of the charged samples was tested in its quiescent state for % Pen and $\Delta P$, and the QF was calculated as described above. These results are reported below as Initial % Pen, Initial $\Delta P$ and Initial QF "Qo".

Accelerated Aging Filtration Performance:

In order to determine the stability of the filtration performance, accelerated aging was tested by comparing the initial QF of charged BMF webs with its QF after storage at an elevated temperature for a specified period of time. The webs are stored for 72 hours (3 days) at 71°C in air. This quality factor after aging at this condition is typically designated as "$Q_3$". The % Charge Retention is calculated by the following equation:

$$\% \text{ Retention} = Q_3 \text{ (after aging for 72 hours at 71°C)} / Q_0 \text{ (initial)} \times 100\%$$

**TIPS Membranes**

The following TIPS membranes were utilized in the examples:

**Film A: A** polyvinylidene fluoride (PVdF) film made as described in U.S. Patent No. 7,338,692 having a 4 micron bubble point pore size, a 5 sec water flux, 125 micrometer thickness, 53% porosity and a 3.2 second Gurley.

**Film B: A** polyvinylidene fluoride (PVdF) film made as described in U.S. Patent No. 7,338,692 having a 1.9 microns bubble point pore size, a 9.4 sec water flux, 125 micrometer thickness, 72% porosity and a 5 second Gurley.

**Film C:** An ethylene-vinyl alcohol copolymer (EVAL $^{(TM)}$) film made as described in U.S. Patent No. 5,962,544 having a 0.76 micron bubble point pore size, a 109 micron thickness (4 mils), 52% porosity and 69 dynes surface wetting energy.

**Nonwoven Materials**

The following nonwoven materials were utilized in the examples:

**Nonwoven A.** An airlaid bicomponent nonwoven (Style 4104 50/50 PE sheath/PET, available from PGI Nonwovens, Charlotte, NC) having a basis weight of 51 grams/meter$^2$ (1.5 ounces/yd$^2$).

**Nonwoven B:** A carded, thermal bonded nonwoven (Novenette Style 149-051, 70% rayon/30% PP, available from FiberWeb Nonwovens, Simpsonville, SC) having a basis weight of 62 grams/meter$^2$.

**Nonwoven C:** A polypropylene blown microfiber (BMF) web prepared with Total 3960 polypropylene resin (Total Petrochemicals, Houston, TX) with a 54 grams per square meter basis weight.

## Foam Material

**Polyurethane foam.** 3M brand TEGAFOAM™ (available from 3M Company, St. Paul, MN). The thickness was approximately 64 mm (0.25 inches).

## Example 1

The PVdF TIPS Film A described above was made hydrophilic by imbibing it with a 10% methanolic solution of SR 344 (PEG 400 diacrylate, available from Sartomer Company, Exton, PA) followed by laminating the film between two sheets of 125 micron polyester (PET) liner and passing it through an electron beam, using a dose of 20 kGy to effect radiation grafting. The electron beam was a model CB-300 made by Energy Sciences of Wilmington, MA and was operated at the maximum voltage of 300 kV and the web was processed through the beam at a speed of 6 meter/min (20 feet/min). The irradiated film was washed with water and dried.

The hydrophilized film was then imbibed with a silver oxide solution. The silver oxide containing solution was prepared by combining 5 parts ammonium carbonate with 95 parts water and mixing until the salt was dissolved. One part silver oxide (AgO) was added to this solution. The mixture was stirred at 60°C for one hour until the silver oxide was dissolved resulting in a clear transparent solution containing silver ions. The imbibed film was laminated and irradiated as above using a dose of 40 kGy. The film was washed in water and dried. The resulting film contained silver metal nanoclusters within the pores of the film.

## Example 2

A silver metal nanocluster containing film was prepared as in Example 1 above except the PVdF TIPS Film B was used as the porous substrate. The resulting film contained silver metal nanoclusters within the pores of the film.

## Example 3

TIPS Film A was hydrophilized as in Example 1 above. Following the hydrophilization step, the film was hydrophilized again by imbibing the film with a methanolic solution of 1% APTAC monomer [3-(acryloylamino)propyl]-trimethylammonium chloride (available as a water solution from Sigma-Aldrich, St. Louis, MO) and was irradiated in the same manner as in Example 1 above using a dose of 40 kGy. The hydrophilized film was then imbibed with a silver nitrate solution. The aqueous silver nitrate solution contained 0.04 M silver nitrate and 0.2 M (12%) isopropanol (IPA). The imbibed film was laminated and irradiated as in Example 1 above using a dose of 40 kGy. The film was washed in water and dried. The resulting film contained silver metal nanoclusters within the pores of the film.

## Example 4

A silver metal nanocluster containing film was prepared as in Example 3 above except the PVdF TIPS Film B was used as the porous substrate. The resulting film contained silver metal nanoclusters within the pores of the film.

## Example 5

A silver metal nanocluster containing film was prepared as in Example 3 above except a methanolic solution of 25% APTAC monomer [3-(acryloylamino)propyl]-trimethylammonium chloride (available as a water solution from Sigma-Aldrich, St. Louis, MO) was used to imbibe the porous substrate in the second hydrophilization step. The imbibed film was laminated and irradiated as in Example 1 above using a dose of 40 kGy. The film was washed in water and dried. The resulting film contained silver metal nanoclusters within the pores of the film.

## Example 6

A silver metal nanocluster containing film was prepared as in Example 5 above except the PVdF TIPS Film B was used as the porous substrate. The resulting film contained silver metal nanoclusters within the pores of the film.

### Example 7

A silver metal nanocluster containing porous substrate was prepared as in Example 1 above except the polyurethane foam described above was used as the porous substrate. The imbibed foam was laminated and irradiated as in Example 1 above using a dose of 40 kGy. The foam was washed in water and dried. The resulting foam contained silver metal nanoclusters within the pores of the foam.

### Example 8

A silver metal nanocluster containing porous substrate was prepared as in Example 1 above except the Nonwoven A described above was used as the porous substrate. The imbibed nonwoven was laminated and irradiated as in Example 1 above using a dose of 70 kGy. The nonwoven was washed in water and dried. The resulting nonwoven contained silver metal nanoclusters within the pores of the nonwoven.

### Comparative Example C1

A nonwoven porous substrate was prepared as in Example 8 above except the silver nitrate imbibing step was not performed. The hydrophilized nonwoven was laminated and irradiated as in Example 8 above using a dose of 70 kGy. The nonwoven was washed in water and dried. The resulting nonwoven did not contain silver metal nanoclusters.

AATCC Method 100 (described above) was used to assess the microbial activity of Examples 1-8 and Comparative Example C1. All samples showed excellent activity against P. aeruginosa (100% reduction) with example 7 showing excellent activity against both organisms tested (100% reduction), and examples 3 and 5 giving moderate activity against S. aureus in addition to 100% reduction of P. aeruginosa. The results are shown in Table 1 below. It is important to note that silver ions in general are very effective against gram negative bacteria such as P. aeruginosa, but moderately effective against gram positive bacteria such as S. aureus.

Table 1: Percent (%) Reduction

|  | **Test Organism** | |
| --- | --- | --- |
|  | S. aureus | P. aeruginosa |
| Example | 24 hours | 24 hours |
| 1 | 78.5 | 100.0 |
| 2 | 59.2 | 100.0 |
| 3 | 96.7 | 100.0 |
| 4 | 70.5 | 100.0 |
| 5 | 95.7 | 100.0 |
| 6 | 78.9 | 100.0 |
| 7 | 100.0 | 100.0 |
| 8 | 79.3 | 100.0 |
| C1 | 48.0 | 100.0 |

To verifiy the formation of silver nano-particles, Tapping Mode Atomic Force Microscopy measurements were conducted. The instrument used for this analysis was a Digital Instruments Dimension 3100 SPM System with a Nanoscope V controller (2008). The probe was an Olympus OTESPA single crystal silicon with a force constant of ~40N/m. The data was analyzed using Nanoscope 5.30r3sr3. AFM images clearly showed the nano-sized silver clusters on BMF fibers. The particles ranged in size between 5-50nm.

### Example 9

A silver metal nanocluster containing porous substrate was prepared as in Example 1 above except the EVAL (™) TIPS Film C described above was used as the porous substrate. A 1% aqueous solution of $AgNO_3$ (0.06 M) was prepared and used to imbibe the film. The imbibed film was laminated and irradiated as in Example 1 using a dose of 40 kGy. After irradiation, each sample was immediately washed twice with water. The resulting film contained silver metal nanoclusters within the pores of the film.

## Example 10

A silver metal nanocluster containing porous substrate was prepared as in Example 9 above except the 1% aqueous solution of AgNO$_3$ was diluted to a 0.2% AgNO$_3$ concentration and used to imbibe the film. The samples were irradiated according to the procedure of Example 1. After irradiation, each sample was immediately washed twice with water. The resulting film contained silver metal nanoclusters within the pores of the film.

## Example 11

A silver metal nanocluster containing porous substrate was prepared as in Example 9 above except the film was irradiated using a dose of 20 kGy. After irradiation, the film was immediately washed twice with water. The resulting film contained silver metal nanoclusters within the pores of the film.

## Example 12

A silver metal nanocluster containing porous substrate was prepared as in Example 10 above except the film was irradiated using a dose of 20 kGy. After irradiation, the film was immediately washed twice with water. The resulting film contained silver metal nanoclusters within the pores of the film.

## Example 13

A silver metal nanocluster containing porous substrate was prepared as in Example 9 above except a 1% AgNO$_3$ (0.06 M) solution in isopropanol was prepared, adding just enough water to dissolve the salt in the isopropanol and used to imbibe the film. The film was irradiated according to the procedure of Example 1 except a dose of 40 kGy was used. After irradiation, the film was immediately washed twice with water. The resulting film contained silver metal nanoclusters within the pores of the film.

## Example 14

A silver metal nanocluster containing porous substrate was prepared as in Example 13 above except the 1% solution of AgNO$_3$ was diluted to a 0.2% AgNO$_3$ concentration and was used to imbibe the film. The film was irradiated according to the procedure of Example 1 using a dose of 40 kGy. After irradiation, each sample was immediately washed twice with water. The resulting film contained silver metal nanoclusters within the pores of the film.

## Example 15

A silver metal nanocluster containing porous substrate was prepared as in Example 13 above except a dose of 20 kGy was used. After irradiation, the film was immediately washed twice with water. The resulting film contained silver metal nanoclusters within the pores of the film.

## Example 16

A silver metal nanocluster containing porous substrate was prepared as in Example 14 above except a dose of 20 kGy. After irradiation, each sample was immediately washed twice with water. The resulting film contained silver metal nanoclusters within the pores of the film.

## Example 17

A silver metal nanocluster containing porous substrate was prepared as in Example 9 above except the PVDF TIPS Film A described above was used as the porous substrate. A 1% aqueous solution of AgNO$_3$ (0.06 M) was prepared and used to imbibe the film. The imbibed film was laminated and irradiated as in Example 1 using a dose of 40 kGy. After irradiation, each sample was immediately washed twice with water. The resulting film contained silver metal nanoclusters within the pores of the film.

**Example 18**

A silver metal nanocluster containing porous substrate was prepared as in Example 17 above except the 1% aqueous solution of $AgNO_3$ was diluted to a 0.2% $AgNO_3$ concentration and used to imbibe the film. The samples were irradiated according to the procedure of Example 1. After irradiation, each sample was immediately washed twice with water. The resulting film contained silver metal nanoclusters within the pores of the film.

**Example 19**

A silver metal nanocluster containing porous substrate was prepared as in Example 17 above except the film was irradiated using a dose of 20 kGy. After irradiation, the film was immediately washed twice with water. The resulting film contained silver metal nanoclusters within the pores of the film.

**Example 20**

A silver metal nanocluster containing porous substrate was prepared as in Example 18 above except the film was irradiated using a dose of 20 kGy. After irradiation, the film was immediately washed twice with water. The resulting film contained silver metal nanoclusters within the pores of the film.

**Example 21**

A silver metal nanocluster containing porous substrate was prepared as in Example 9 above except a 1 % $AgNO_3$ (0.06 M) solution in isopropanol was prepared, adding just enough water to dissolve the salt in the isopropanol and used to imbibe the film. The film was irradiated according to the procedure of Example 1 except a dose of 40 kGy was used. After irradiation, the film was immediately washed twice with water. The resulting film contained silver metal nanoclusters within the pores of the film.

**Example 22**

A silver metal nanocluster containing porous substrate was prepared as in Example 21 above except the 1% solution of $AgNO_3$ was diluted to a 0.2% $AgNO_3$ concentration and was used to imbibe the film. The film was irradiated according to the procedure of Example 1 using a dose of 40 kGy. After irradiation, each sample was immediately washed twice with water. The resulting film contained silver metal nanoclusters within the pores of the film.

**Example 23**

A silver metal nanocluster containing porous substrate was prepared as in Example 21 above except a dose of 20 kGy was used. After irradiation, the film was immediately washed twice with water. The resulting film contained silver metal nanoclusters within the pores of the film.

**Example 24**

A silver metal nanocluster containing porous substrate was prepared as in Example 22 above except a dose of 20 kGy. After irradiation, the film was immediately washed twice with water. The resulting film contained silver metal nanoclusters within the pores of the film.

AATCC Method 100 (described above) was used to assess the microbial activity of Examples 9-24. The results are shown in Table 2. A negative value for percent reduction indicates that the number of colony forming units per $cm^2$ grew in number. When zone of inhibition testing was done on these samples, trypticase soy agar (TSA) was used instead of Mueller-Hinton II. The TSA binds silver ions preventing their diffusion through the agar, and effectively eliminating the formation of a zone. All samples tested showed no growth under the sample disks for both types of organisms tested indicating that the silver nanoparticles are effective in preventing bacteria growth under the sample.

Table 2. Percent (%) Reduction.

| Example | Microorganism |
|---|---|
|  | S. aureus |
| 9 (1% silver/40 kGy) | 53 |
| 10 (0.2% silver/40 kGy) | -26 |

(continued)

| Example | Microorganism S. aureus |
|---|---|
| 11 (1% silver/20 kGy) | -32 |
| 12 (0.2% silver/20 kGy) | 60 |
| 13 (1% silver/40 kGy) | 66 |
| 14 (0.2% silver/40 kGy) | -83 |
| 15 (1% silver/20 kGy) | -36 |
| 16 (0.2% silver/20 kGy) | -6 |
| 17 (1% silver/40 kGy) | 100 |
| 18 (0.2% silver/40 kGy) | 100 |
| 19 (1% silver/20 kGy) | 100 |
| 20 (0.2% silver/20 kGy) | 93 |
| 21 (1% silver/40 kGy) | 100 |
| 22 (0.2% silver/40 kGy) | 100 |
| 23 (1% silver/20 kGy) | 100 |
| 24 (0.2% silver/20 kGy) | 100 |

### Example 25

A silver metal nanocluster containing porous substrate was prepared as in Example 1 except the film was imbibed with an aqueous solution containing 0.2% $AgNO_3$ and 12% IPA. The imbibed film was irradiated following the procedure of Example 1 using a dose of 30 kGy. After irradiation, the film was immediately washed twice with water. The resulting film contained silver metal nanoclusters within the pores of the film.

### Example 26

A silver metal nanocluster containing porous substrate was prepared as in Example 1 except the substrate was a piece of cotton gauze and was imbibed with an aqueous solution containing 1% $AgNO_3$ and 12% IPA. The imbibed film was irradiated following the procedure of Example 1 using a dose of 40 kGy. After irradiation, the cotton gauze was immediately washed twice with water. The resulting film contained silver metal nanoclusters within the pores of the gauze.

### Example 27

A silver metal nanocluster containing porous substrate was prepared as in Example 1 except the substrate was a polyurethane foam as described above. The foam was imbibed with an aqueous solution containing 1% $AgNO_3$ and 12% IPA on only one surface of the foam. To ensure good uniformity and minimum absorption of solution into the foam, hydrophobic PVdF TIPS Film B was imbibed and placed on top of the foam and then irradiated with a dose of 40 kGy. The foam was then immediately washed, releasing a large quantity of nanosilver particles.

Table 3: Percent (%) Reduction

| Example | Microorganism | |
|---|---|---|
| | S. aureus | Ps. Aeruginosa |
| 25 | 94 | 100 |
| 26 | 83 | 100 |
| 27 | 96 | 100 |

In order to determine the total amount of silver deposited on each porous substrate, Inductively Coupled Plasma - Atomic Emission Spectroscopy (ICP-AES) was employed. The results are shown in Table 4 below. The samples appeared non-homogenous giving significantly different silver concentrations at different regions of the substrate. Silver concentration in the tested samples ranged between 1000 ppm and 5000 ppm.

Table 4: ICP-AES results

| Example | Ag concentration (ppm) | Sample Mass (g) | Sample Area (in x in) |
|---|---|---|---|
| 25 | 4600, 2000 | 0.1552, 0.1296 | 3x2.5, 3x1.44 |
| 26 | 2600 | 0.1601 | 3x3 |
| 27 | 980, 1100 | 1.6362, 1.4451 | 3x1.56, 3x1.44 |

Zone of inhibition testing was conducted on those samples using Mueller-Hinton agar. 8mm diameter circular samples were cut from the above samples using a dye. Two circles of each sample were placed on each plate. Each sample was tested against S.aureus and P.aeruginosa. After 24 hours in a 37° C incubator, the plates were removed and the zone of clearance measured for the samples as shown in Table 5 below.

Table 5: Zone of Inhibition

| | Zones Against (mm) | | Avg. Zone against (mm) | |
|---|---|---|---|---|
| Example | S.aureus | P.aeruginosa | S.aureus | P.aeruginosa |
| 25 | 14 | 12,11 | 14 | 11.5 |
| 26 | 12 | 14 | 12 | 14 |
| 27 | 13,12 | 15,15 | 12.5 | 15 |

As can be seen from the data in Table 5, when using Mueller-Hinton agar, good size zones of no growth are observed with the nanosilver containing substrates.

### Example 28

A gold metal nanocluster containing porous substrate was prepared as in Example 1 except the substrate was a PVDF TIPS Film B described above. A 2% gold chloride solution was prepared by dissolving 1 part gold chloride in 50 parts distilled water. The gold chloride dissolved readily resulting in a clear colorless solution. A small amount of IPA was added to boost the effectiveness of nanoparticle formation. The film was imbibed with the gold chloride solution, laminated and irradiated with a dose of 40 kGy following the procedure of Example 1. After passing through the beam, the sample slowly developed the magenta tint of nano-gold particles in the film. Based on the color, the size of the nanoparticles was estimated to be around 10 nm.

### Example 29

A gold metal nanocluster containing porous substrate was prepared as in Example 28 except the substrate was an EVAL(™) TIPS Film C described above.

### Example 30

A copper metal nanocluster containing porous substrate was prepared as in Example 1 except the substrate was a PVDF TIPS Film A described above. A dilute solution of copper sulfate was made up in 12% aqueous IPA, 50 mg into 50 ml of solution. The film was imbibed and laminated between two sheets of polyester film, and then irradiated using a dose of 40 kGy following the procedure of Example 1. The sample was a distinct copper brown in color upon exiting the beam and remained so for several minutes before oxygen diffusion rendered the membrane completely white again as the nanocopper oxidized.

Another film sample was similarly processed but was imbibed within an inert atmosphere glove box. The film was irradiated in a ZIPLOC™ bag for processing through the beam and then returned to the glove box. After several days in an atmosphere of only several ppm of oxygen, the sample continued to show the presence of nanocopper (not oxidized) and remained brown.

### Example 31

A silver metal nanocluster containing porous substrate was prepared as in Example 1 except the substrate was nonwoven substrate C.

**Electret Charging Process:**

The BMF webs used in Examples 31-33 were hydrocharged using the following technique. A fine spray of high purity water having a conductivity of less than 5 microS/cm was continuously generated from a nozzle operating at a pressure of 896 kiloPascals (130 psig) and a flow rate of approximately 1.4 liters/minute. The BMF web was conveyed by a porous belt through the water spray at a speed of approximately 10 centimeters/second while a vacuum simultaneously drew the water through the web from below. The BMF web was run through the sprayer twice (sequentially once on each side) and then allowed to dry completely overnight prior to filter testing.

An antimicrobial solution was prepared by dissolving 0.2 parts of silver sulfate in a 25% IPA/75% water solution. The resulting saturated solution of silver sulfate (less than 0.2 wt %) was used to imbibe a 61 cm long and 30 cm wide sample of the charged BMF web. The imbibed web was blotted of excess liquid and sandwiched between two sheets of PET. The sandwich was electron-beam irradiated with a dose of 20 kGy at an accelerating voltage of 300 keV. A large quantity of nano-silver clusters was generated within the BMF web. The BMF web was lightly blotted and then rinsed with water. Some of the nano-silver was leached and redistributed to achieve a golden color all the way through.

## Example 32

A silver metal nanocluster containing porous substrate was prepared as in Example 31 except the BMF nonwoven substrate was not charged. The BMF nonwoven was immediately rinsed in water and a large volume of yellow-brown color was initially leached out into the wash water but appeared to resettle into the BMF web to create a more uniform distribution within the BMF web.

## Example 33

A silver metal nanocluster containing porous substrate was prepared as in Example 32 except the imbibed BMF web was blotted immediately with paper towels. A large quantity of liquid was absorbed out of the BMF web and the paper towels were darkened with nano-silver leaving the BMF web lighter in color as a result.

The pressure drop across the web ($\Delta P$) along with the Quality Factor of the webs (an indication of filtration efficiency) were measured for Examples 31-33. The uncharged samples were hydrocharged after E-beam treatment but prior to Quality Factor measurement (Q0). Examples 32 and 33 were aged in an oven set at 71°C for 3 days, and the Quality Factor (Q3) after aging was measured to assess charge retention of the treated samples. The results are shown in Table 6.

Table 6: Quality Factor

| | Q0 Quality Factor | | | Q3 Quality Factor | | |
|---|---|---|---|---|---|---|
| Example | $\Delta P$ | Pen% | Q0 | $\Delta P$ | Pen% | Q3 |
| 31 | 2.45 | 21.00 | 0.64 | | | |
| 32 | 1.90 | 46.35 | 0.41 | 1.90 | 55.40 | 0.31 |
| 33 | 1.85 | 28.95 | 0.67 | 1.80 | 40.55 | 0.50 |

Antimicrobial performance of Examples 31-33 both before and after aging are shown in Table 7. All three examples resulted in a greater than 2 log reduction of microbial load, and the washed samples resulted in a greater than 4 log reduction. Oven aging did not have an effect on antimicrobial performance of the webs.

Table 7: Antimicrobial activity Against *Pseudomonas aeruginosa*

| Example | 0 minutes (CFU/cm$^2$) | 24 hours (CFU/cm$^2$) | % Redaction |
|---|---|---|---|
| 31: charged BMF then E-beamed, blot, wash | 6.88E+04 | 1.39E+02 | 99.8 |
| 32: uncharged BMF blotted dry | 6.37E+04 | 1.18E+02 | 99.8 |
| 32: uncharged BMF blotted dry (aged) | 5.64E+04 | 1.64E+02 | 99.7 |
| 33: uncharged BMF washed | 6.50E+04 | 1.27E+00 | 100 |
| 33: uncharged BMF washed (aged) | 5.86E+04 | <5.1 | 100 |

Various modifications and alterations to this invention will become apparent to those skilled in the art without departing from the scope and spirit of this invention. It should be understood that this invention is not intended to be unduly limited by the illustrative embodiments and examples set forth herein and that such examples and embodiments are presented by way of example only with the scope of the invention intended to be limited only by the claims set forth herein as follows.

**Claims**

1. A continuous method for *in situ* formation of metal nanoclusters within a porous substrate comprising:

   (a) imbibing a porous substrate with an aqueous heavy metal salt solution; and
   (b) exposing the imbibed porous substrate to ionizing radiation to form heavy metal nanoclusters within the porous substrate,

   wherein the method is carried out without any vacuum processing and the porous substrate is a fabric or a microporous substrate comprising one or more polymeric materials.

2. The method of claim 1 wherein the porous substrate has pores or interstices having an average size of less than 100 $\mu$m.

3. The method of claim 1 or claim 2 wherein the porous substrate is a thermally induced phase-separated membrane.

4. The method of claim 3 wherein the thermally induced phase-separated membrane has pores having an average size of less than about 10 $\mu$m, and wherein the thermally induced phase-separated membrane is hydrophobic.

5. The method of claim 1 wherein the porous substrate is a charged knit, braided, woven or non-woven fabric and wherein the porous knit, braided, woven or non-woven fabric remains charged after the exposing step (b).

6. The method any of claims 1 to 5 wherein the average size of the heavy metal nanoclusters is less than about 100 nm.

7. The method of any of claims 1 to 6 wherein exposing the aqueous heavy metal salt solution to ionizing radiation comprises using an electron-beam.

8. The method of any of claims 1 to 7 further comprising drying the imbibed porous substrate after exposing the aqueous heavy metal salt solution to ionizing radiation.

9. The method of any of claims 1 to 7 further comprising washing the imbibed porous substrate with solvent.

**Patentansprüche**

1. Kontinuierliches Verfahren zur *in situ* Bildung metallischer Nanocluster innerhalb eines porösen Trägermaterials, das Folgendes umfasst:

   (a) Tränken eines porösen Trägermaterials mit einer wässrigen Schwermetallsalzlösung; und
   (b) Aussetzen des getränkten porösen Trägermaterials gegenüber ionisierender Strahlung, um Schwermetallnanocluster innerhalb des porösen Trägermaterials zu bilden,

   wobei das Verfahren ohne Vakuumverarbeitung ausgeführt wird und das poröse Trägermaterial ein Gewebe oder ein mikroporöses Trägermaterial ist, das ein oder mehrere polymere Materialien umfasst.

2. Verfahren nach Anspruch 1, wobei das poröse Trägermaterial Poren oder Zwischenräume aufweist, die eine durchschnittliche Größe von weniger als 100 $\mu$m aufweisen.

3. Verfahren nach Anspruch 1 oder Anspruch 2, wobei das poröse Trägermaterial eine thermisch induzierte Membran mit getrennten Phasen ist.

4. Verfahren nach Anspruch 3, wobei die thermisch induzierte Membran mit getrennten Phasen Poren aufweist, die eine durchschnittliche Größe von weniger als etwa 10 μm aufweisen und wobei die thermisch induzierte Membran mit getrennten Phasen hydrophob ist.

5. Verfahren nach Anspruch 1, wobei das poröse Trägermaterial ein geladenes gestricktes, geflochtenes, gewobenes oder Vliesgewebe ist und wobei das poröse gestrickte, geknüpfte, gewobene oder Vliesgewebe nach dem Schritt (b) des Aussetzens geladen bleibt.

6. Verfahren nach einem der Ansprüche 1 bis 5, wobei die durchschnittliche Größe der Schwermetallnanocluster weniger als etwa 100 nm ist.

7. Verfahren nach einem der Ansprüche 1 bis 6, wobei das Aussetzen der wässrigen Schwermetallsalzlösung gegenüber ionisierender Strahlung die Verwendung eines Elektronenstrahls umfasst.

8. Verfahren nach einem der Ansprüche 1 bis 7, das ferner das Trocknen des getränkten porösen Trägermaterials nach dem Aussetzen der wässrigen Schwermetallsalzlösung gegenüber ionisierender Strahlung umfasst.

9. Verfahren nach einem der Ansprüche 1 bis 7, das ferner das Waschen des getränkten porösen Trägermaterials mit Lösemittel umfasst.

**Revendications**

1. Procédé en continu pour la formation *in situ* de nanoagrégats de métal à l'intérieur d'un substrat poreux, comprenant :

   (a) l'imbibition d'un substrat poreux avec une solution aqueuse de sel de métal lourd ; et
   (b) l'exposition du substrat poreux imbibé à un rayonnement ionisant pour former des nanoagrégats de métal lourd à l'intérieur du substrat poreux,

   le procédé étant exécuté sans aucun traitement sous vide et le substrat poreux étant un tissu ou un substrat microporeux comprenant un ou plusieurs matériaux polymères.

2. Procédé selon la revendication 1, dans lequel le substrat poreux comporte des pores ou des interstices ayant une taille moyenne inférieure à 100 μm.

3. Procédé selon la revendication 1 ou la revendication 2, dans lequel le substrat poreux est une membrane à séparation de phase induite thermiquement.

4. Procédé selon la revendication 3, dans lequel la membrane à séparation de phase induite thermiquement comporte des pores ayant une taille moyenne inférieure à environ 10 μm, et dans lequel la membrane à séparation de phase induite thermiquement est hydrophobe.

5. Procédé selon la revendication 1, dans lequel le substrat poreux est un tissu chargé tricoté, tressé, tissé ou non tissé et dans lequel le tissu poreux tricoté, tressé, tissé ou non tissé reste chargé après l'étape d'exposition (b).

6. Procédé selon l'une quelconque des revendications 1 à 5, dans lequel la taille moyenne des nanoagrégats de métal lourd est inférieure à environ 100 nm.

7. Procédé selon l'une quelconque des revendications 1 à 6, dans lequel l'exposition de la solution aqueuse de sel de métal lourd à un rayonnement ionisant comprend l'utilisation d'un faisceau d'électrons.

8. Procédé selon l'une quelconque des revendications 1 à 7, comprenant en outre le séchage du substrat poreux imbibé après l'exposition de la solution aqueuse de sel de métal lourd au rayonnement ionisant.

9. Procédé selon l'une quelconque des revendications 1 à 7, comprenant en outre le lavage du substrat poreux imbibé avec un solvant.

*FIG. 1*

# EP 2 303 770 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007044590 A
- JP 2005298993 A
- US 1529256 A
- US 4539256 A
- US 4726989 A
- US 4867881 A
- US 5120594 A
- US 5260360 A
- US 5962544 A
- US 20050058821 A, Smith
- US 7338692 B

**Non-patent literature cited in the description**

- *Chem. Mater.,* 2003, vol. 15, 4401-4406
- *Radiation Physics and Chemistry,* 2007, vol. 76, 1290-1294
- *Chem. Mater.,* 2003, vol. 15, 1993-1999
- **WENTE, V.A.** Superfine Thermoplastic Fibers. *Industrial Engineering Chemistry,* 1956, vol. 48, 1342-1346
- **WENTE, V.A.** Manufacture of Super Fine Organic Fibers. *Naval Research Laboratories (Report No. 4364),* 25 May 1954